# EUROPEAN PATENT APPLICATION

(11) **EP 4 631 973 A1**
(43) Date of publication of application: **15.10.2025**
(21) Application number: 23907856.1
(22) Date of filing: 22.12.2023
(51) Int. Cl.: C07K 14/605, C07K 14/00, A61P 3/04, A61K 38/00

(54) **NOVEL TRIPLE ACTIVATOR HAVING ACTIVITY ON ALL OF GLP-1, GIP, AND GLUCAGON RECEPTORS, AND PHARMACEUTICAL COMPOSITION FOR PREVENTING OR TREATING OBESITY COMPRISING SAME**

(30) Priority: 23.12.2022 KR 20220182980
(71) Applicant: Hanmi Pharm. Co., Ltd., Hwaseong-si, Gyeonggi-do 18536 (KR)
(72) Inventor: KIM, Yo Han, Hwaseong-si, Gyeonggi-do 18469 (KR); KIM, Jung Kuk, Hwaseong-si, Gyeonggi-do 18469 (KR); SHIN, Min Kyung, Hwaseong-si, Gyeonggi-do 18469 (KR); IM, Hyeon Joo, Hwaseong-si, Gyeonggi-do 18469 (KR)
(74) Representative: Mewburn Ellis LLP
(86) International application number: PCT/KR2023/021392
(87) International publication number: WO 2024/136573

(57) **Abstract**

The present disclosure relates to a novel triple agonist having activities for all of GLP-1, GIP, and glucagon receptors, and use of the triple agonist for the prevention or treatment of obesity.

## Description

### [Technical Field]

The present disclosure relates to a triple agonist having activities for all of GLP-1, GIP, and glucagon receptors and use thereof.

### [Background Art]

Obesity is a representative metabolic disease that occurs in modern society, and is regarded as a health-threatening factor in the world because it may lead to various diseases, and the accompanying economic costs due to the incidence of the disease are rapidly increasing at present.

Glucagon-like peptide-1 (GLP-1) and glucose-dependent insulinotropic polypeptide (GIP) are representative gastrointestinal hormones and neuronal hormones, and are materials involved in the control of blood glucose levels according to food intake. GLP-1 is a hormone secreted by the small intestine stimulated by food intake, and promotes insulin secretion in the pancreas in a blood glucose-dependent manner and inhibits the secretion of glucagon, thus helping the action of lowering blood glucose levels.

Additionally, GIP, one of the gastrointestinal hormones secreted by the stimulation of food intake, as is the case of GLP-1, is a hormone consisting of 42 amino acids secreted by the intestinal K-cells. It has been reported that GIP performs the functions of promoting the secretion of insulin in the pancreas in a blood glucose-dependent manner and helping to lower the blood glucose levels, and exhibits the effects of increasing the activation of GLP-1, anti-inflammation, *etc.*

Glucagon is a peptide hormone secreted by the pancreas, and is involved in the control of blood glucose levels together with the two materials mentioned above. Glucagon is produced in the pancreas when the blood glucose levels fall due to reasons such as medications, diseases, deficiency in hormones or enzymes, *etc.* Glucagon sends a signal for glycogen breakdown in the liver to induce the release of glucose and increases blood glucose levels to a normal level. In addition to the effect of increasing the blood glucose levels, glucagon suppresses appetite in animals and humans and activates hormone-sensitive lipase of adipocytes to promote lipolysis and energy expenditure, thereby showing an anti-obesity effect.

The need has recently emerged for a material that can act simultaneously on the above receptors to increase efficacy or improve side effects, compared to the existing single agonists for the GLP-1 receptor, GIP receptor, or glucagon receptor, and the present inventors have developed peptides that can act on the GLP-1, GIP, and glucagon receptors, and conjugates thereof (WO 2017-116204; WO 2017-116205).

### [Disclosure]

### [Technical Problem]

In addition to the previously known triple agonists, efforts are continuously being made to develop a triple agonist having activities for all of GLP-1, GIP, and glucagon receptors with novel structures and sequences as an excellent obesity therapeutic agent.

### [Technical Solution]

It is one object of the present disclosure to provide a peptide having activities for a glucagon-like peptide-1 (GLP-1) receptor, a glucose-dependent insulinotropic polypeptide (GIP) receptor, and a glucagon receptor.

It is another object of the present disclosure to provide a pharmaceutical composition for preventing or treating obesity, including the peptide.

It is still another object of the present disclosure to provide a method for preventing or treating obesity, including: administering the peptide or the pharmaceutical composition to a subject in need.

It is yet another object of the present disclosure to provide the use of the peptide or the pharmaceutical composition for the prevention or treatment of obesity.

It is even another object of the present disclosure to provide the use of the peptide or the pharmaceutical composition in the preparation of a medicament for the prevention or treatment of obesity.

### [Advantageous Effects]

The peptide having activities for a glucagon-like peptide-1 (GLP-1) receptor, a glucose-dependent insulinotropic polypeptide (GIP) receptor, and a glucagon receptor activates the three receptors simultaneously and thus can exhibits an excellent effect for preventing or treating obesity.

### [Brief Description of Drawings]

FIG. 1 is a diagram showing the change in body weight upon administration of the triple agonists of SEQ ID NOS: 1, 2, and 4 according to the present disclosure and the change in body weight relative to immediately before drug administration on the 14th day.
FIG. 2 is a diagram showing the change in body weight upon administration of the triple agonists of SEQ ID NOS: 3, 5, 6, and 7 according to the present disclosure and the change in body weight relative to immediately before drug administration on the 14th day.

### [Best Mode]

One aspect of the present disclosure provides a novel peptide having activities for a glucagon-like peptide-1 (GLP-1) receptor, a glucose-dependent insulinotropic polypeptide (GIP) receptor, and a glucagon receptor.

In one embodiment, the peptide is characterized in that it is represented by General Formula 1 below:

X1-Aib-X3-G-T-F-T-S-D-Y-S-X12-X13-L-D-E-X17-X18-A-K-X21-F-V-Q-W-L-L-D-X29-H-P-S-S-G-Q-P-P-P-S (General Formula 1, SEQ ID NO: 29)

In General Formula 1 above,
X1 is histidine or tyrosine;
X3 is glutamine or histidine;
X12 is lysine or an acylated amino acid;
X13 is α-methyl-leucine, tyrosine, or alanine;
X17 is lysine or an acylated amino acid;
X18 is alanine or arginine;
X21 is aspartic acid or glutamic acid; and
X29 is histidine or glutamine;
wherein - represents a peptide bond, and the peptide does not contain cysteine.

In another embodiment, the peptide is characterized in that:
in General Formula 1 above, X13 is tyrosine; and X18 is alanine.

In still another embodiment, the peptide is characterized in that:
in General Formula 1 above, X1 is tyrosine; X3 is glutamine; X13 is tyrosine; and X18 is arginine.

In yet another embodiment, the peptide is characterized in that:
in General Formula 1 above, X3 is histidine.

In further another embodiment, the peptide is characterized in that:
in General Formula 1 above, X29 is glutamine.

In still further another embodiment, the peptide is characterized in that it has the structure of General Formula 2 below:

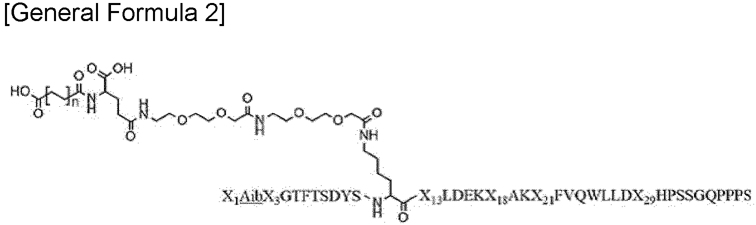

In General Formula 2 above,
n is 16 or 18;
X1 is histidine or tyrosine;
X3 is glutamine or histidine;
X13 is α-methyl-leucine, tyrosine, or alanine;
X18 is alanine or arginine;
X21 is aspartic acid or glutamic acid; and
X29 is histidine or glutamine.

As the peptide according to any one of the above-described embodiments, the peptide has higher relative activities for the GLP-1 receptor and GIP receptor relative to native GLP-1 and GIP, respectively, compared to the relative activity for the glucagon receptor relative to a native glucagon.

As the peptide according to any one of the above-described embodiments, the peptide has 4 times or higher relative activities for the GLP-1 receptor and GIP receptor relative to a native GLP-1 and GIP, respectively, compared to the relative activity for the glucagon receptor relative to a native glucagon.

As the peptide according to any one of the above-described embodiments, an acyl group is attached to one or more amino acids of the peptide directly or through a linker.

As the peptide according to any one of the above-described embodiments, the linker is from a group consisting of (2-(2-aminoethoxy)ethoxy)acetic acid (AEEA), 4-Aminobutyric acid (GABA), 5-Aminovaleric acid (Ava), Aminohexanoic acid (Ahx), triazole, and polyethylene glycol (PEG).

As the peptide according to any one of the above-described embodiments, the linker includes (2-(2-aminoethoxy)ethoxy)acetic acid (AEEA).

As the peptide according to any one of the above-described embodiments, the linker is gammaGlu-(AEEA)₂.

As the peptide according to any one of the above-described embodiments, the linker includes 0 to 3 AEEA, and 0 to 3 gamma-glutamate are linked to AEEA.

As the peptide according to any one of the above-described embodiments, the acylated amino acid is any one amino acid represented by K(1) or K(2) below:
[K(1)] C20diacid-gammaGlu-(AEEA)₂-Lys 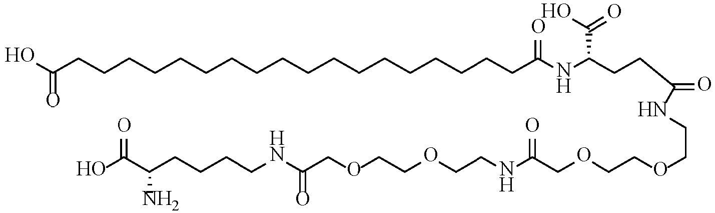
[K(2)] C18diacid-gammaGlu-(AEEA)₂-Lys

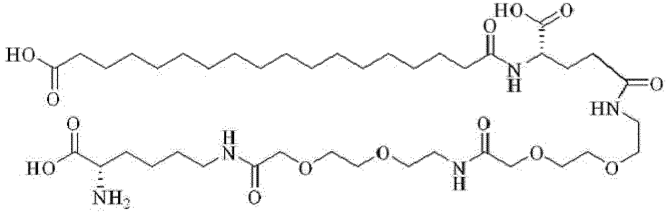

As the peptide according to any one of the above-described embodiments, the peptide is amidated at the C-terminus.

As the peptide according to any one of the above-described embodiments, the peptide is acylated with a C₁ to C₃₀ straight-chain or branched-chain acyl group containing one or two carboxylic acids.

As the peptide according to any one of the above-described embodiments, the acyl group is a C₄ to C₃₀ fatty acid or dicarboxylic acid.

As the peptide according to any one of the above-described embodiments, the peptide is acylated on an amino acid located at the N-terminus or C-terminus or lysine residue.

As the peptide according to any one of the above-described embodiments, the peptide includes any one sequence selected from the group consisting of amino acid sequences of SEQ ID NOS: 1 to 28.

As the peptide according to any one of the above-described embodiments, the amino acids at positions 16 and 20 from the N-terminus form a ring with each other.

As the peptide according to any one of the above-described embodiments, the peptide has any one structure from (i) to (iv) below:

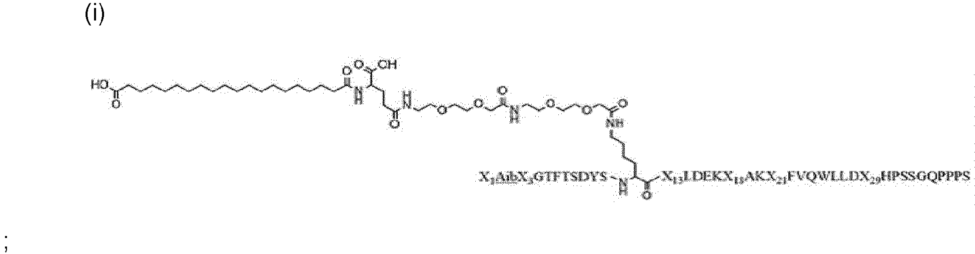

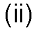

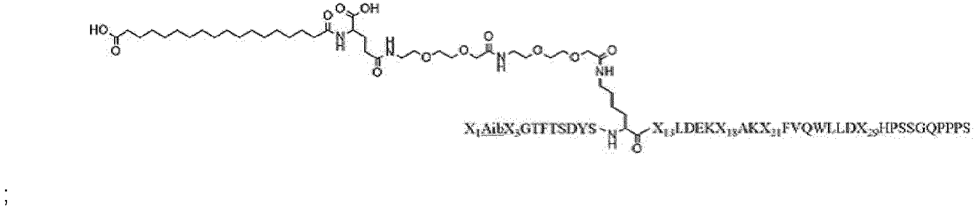

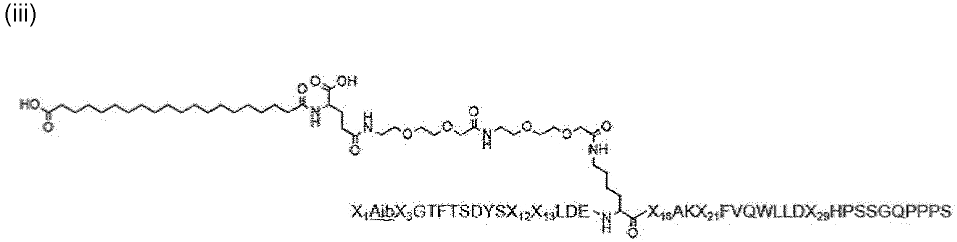

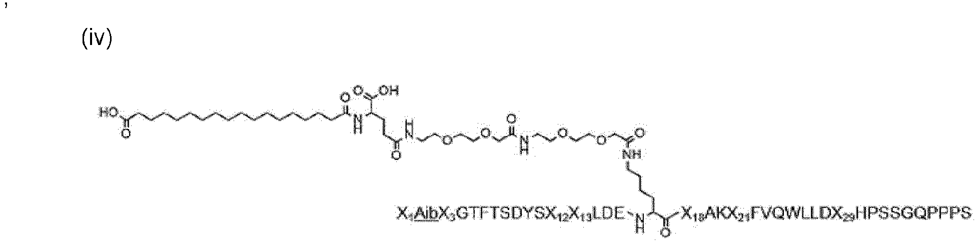

Another aspect of the present disclosure provides a pharmaceutical composition for preventing or treating obesity, including the peptide in a pharmaceutically effective amount.

In one embodiment, the pharmaceutical composition further includes a pharmaceutically acceptable carrier.

In another embodiment, the pharmaceutical composition reduces the body weight of an individual upon administration.

As the pharmaceutical composition according to any one of the above-described embodiments, the pharmaceutical composition includes the peptide having any one structure from (i) to (iv) below:

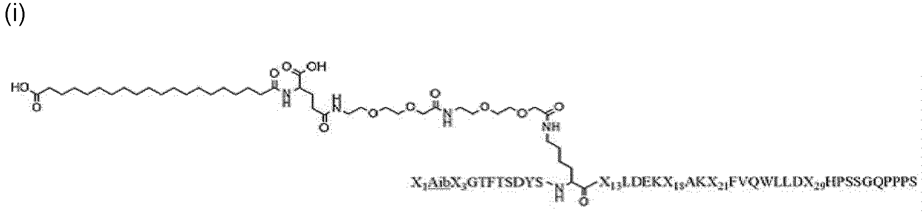

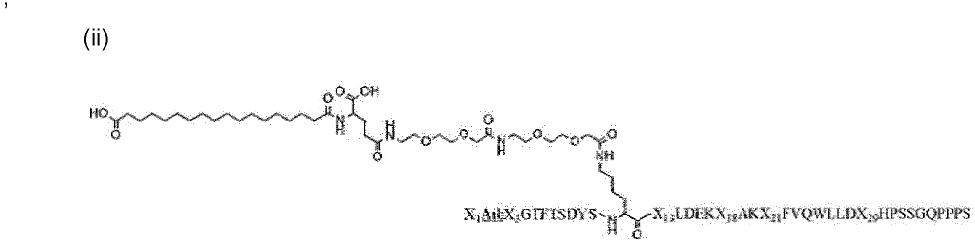

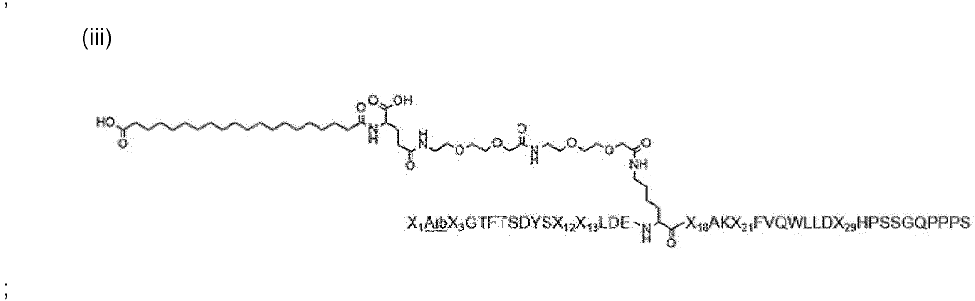

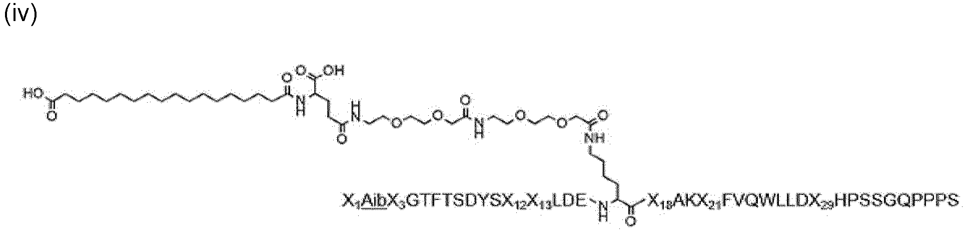

As the pharmaceutical composition according to any one of the above-described embodiments, the pharmaceutical composition includes the peptide having the structure of General Formula 2 below:

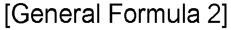

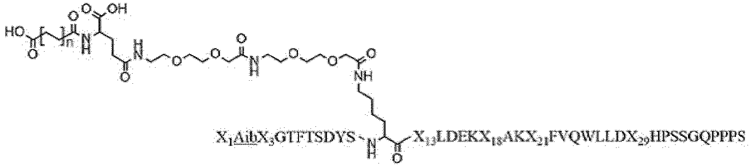

In General Formula 2 above,
n is 16 or 18;
X1 is histidine or tyrosine;
X3 is glutamine or histidine;
X13 is α-methyl-leucine, tyrosine, or alanine;
X18 is alanine or arginine;
X21 is aspartic acid or glutamic acid; and
X29 is histidine or glutamine.

Still another aspect of the present disclosure provides the use of the peptide or the composition including the same for the prevention or treatment of obesity.

Yet another aspect of the present disclosure provides the use of the peptide or the composition including the same in the preparation of a medicament for the prevention or treatment of obesity.

### [DETAILED DESCRIPTION OF THE INVENTION]

Hereinafter, the present disclosure will be described in more detail. Meanwhile, each description and embodiment disclosed herein can be applied to other descriptions and embodiments, respectively. That is, all combinations of various elements disclosed herein fall within the scope of the present disclosure. Further, the scope of the present disclosure is not limited by the specific description described below.

Throughout the entire specification of the present disclosure, not only the conventional one-letter and three-letter codes for naturally occurring amino acids, but also those three-letter codes generally allowed for other amino acids, such as α-aminoisobutyric acid (Aib), Sar (N-methylglycine), α-methyl-glutamic acid, and α-methyl-leucine (αMeL), *etc.* are used. Additionally, the amino acids mentioned herein are abbreviated according to the nomenclature rules of IUPAC-IUB as follows:

| | |
|---|---|
| alanine (A) | arginine (R) |
| asparagine (N) | aspartic acid (D) |
| cysteine (C) | glutamic acid (E) |
| glutamine (Q) | glycine (G) |
| histidine (H) | isoleucine (I) |
| leucine (L) | lysine (K) |
| methionine (M) | phenylalanine (F) |
| proline (P) | serine (S) |
| threonine (T) | tryptophan (W) |
| tyrosine (Y) | valine (V) |

One aspect of the present disclosure provides a peptide having activities for a glucagon-like peptide-1 (GLP-1) receptor, a glucose-dependent insulinotropic polypeptide (GIP) receptor, and a glucagon receptor. The peptide having activities for GLP-1, GIP, and glucagon receptors, or the peptide may be used interchangeably with a triple agonist.

Such a triple agonist includes various materials which have a significant level of activities for GLP-1, GIP, and glucagon receptors (e.g., various peptides).

Although not particularly limited, the triple agonist having a significant level of activities for GLP-1, GIP, and glucagon receptors may exhibit *in vitro* activities which are about 0.1% higher, about 1% higher, about 2% higher, about 3 % higher, about 4% higher, about 5% higher, about 6% higher, about 7% higher, about 8% higher, about 9% higher, about 10 % higher, about 20% higher, about 30% higher, about 40% higher, about 50 % higher, about 60% higher, about 70% higher, about 80% higher, about 90 % higher, and about 100% higher, to one or more receptors, specifically two or more receptors, and more specifically all three of the receptors among the GLP-1, GIP, and glucagon receptors, compared to native ligands of the corresponding receptors (native glucagon, native GLP-1, and native GIP).

Meanwhile, the triple agonist according to the present disclosure is characterized in that it exhibits one or more activities from i) to iii) below, in particular a significant activity:
i) activation of GLP-1 receptor; ii) activation of GIP receptor; and iii) activation of glucagon receptor.

In particular, the activation of the receptors may include, for example, those cases where the *in vitro* activities are about 0.1% higher, about 1% higher, about 2% higher, about 3 % higher, about 4% higher, about 5% higher, about 6% higher, about 7% higher, about 8% higher, about 9% higher, about 10 % higher, about 20% higher, about 30% higher, about 40% higher, about 50 % higher, about 60% higher, about 70% higher, about 80% higher, about 90 % higher, and about 100% higher compared to native ligands, but the activities are not limited thereto.

As used herein, the term "about" refers to a range including all of ± 0.5, ± 0.4, ± 0.3, ± 0.2, ± 0.1, *etc.,* and it includes all of the values equivalent to those which come immediately after the term "about" or those in a similar range, but is not limited thereto.

The activity of the triple agonist may be measured by methods known in the art and is not limited to a specific method. In one example, it may be measured in the same manner as disclosed in Experimental Example 1, but the method is not limited thereto.

The triple agonist of the present disclosure has activities for all of GLP-1, GIP, and glucagon receptors, but the ratio between the relative activities for each receptor may vary. Specifically, the triple agonist according to the present disclosure may have higher relative activities for the GLP-1 receptor and GIP receptor relative to native GLP-1 and GIP, compared to the relative activity for the glucagon receptor relative to native glucagon. The degree of activity for each receptor is important in determining the *in vivo* activity and efficacy of the triple agonist, and the triple agonist of the present disclosure exhibits higher activities for GLP-1 and GIP receptors and thus is effective in the treatment of obesity, *etc.,* and exhibits activity for glucan receptor at the same time, and thus, a synergistic effect may be expected in the treatment of obesity.

Specifically, the triple agonist may have a relative activity for the GLP-1 receptor relative to native GLP-1, which is 4 times or higher, 5 times or higher, 10 times or higher, 15 times or higher, 20 times or higher, or 25 times or higher, or 30 times or lower, compared to the relative activity for the glucagon receptor relative to native glucagon, but is not limited thereto. Additionally, the triple agonist may have a relative activity for the GIP receptor relative to native GIP, which is 4 times or higher, 10 times or higher, 15 times or higher, or 20 times or lower, compared to the relative activity for the glucagon receptor relative to native glucagon, but is not limited thereto.

Based on the relative activity of the triple agonist for the glucagon receptor relative to natural glucagon (a), the relative activity for the GLP-1 receptor relative to native GLP-1 (b); and the relative activity for the GIP receptor relative to native GIP (c) (i.e., (b)/(a); and (c)/(a)) may each independently exhibit at different ratios, and more specifically, the relative activities for the GIP receptor and GLP-1 receptor relative to native GIP and GLP-1 may be higher, compared to the relative activity for the glucagon receptor relative to native glucagon. Although not limited, the triple agonist may have 4 times or higher relative activities for the GIP receptor and GLP-1 receptor relative to native GIP and GLP-1, respectively, compared to the relative activity for the glucagon receptor relative to native glucagon.

Specific examples may be those including the amino acid sequence of any one of SEQ ID NOS: 1, 3, 6, and 9, or those consisting (essentially) of the amino acid sequence of any one of SEQ ID NOS: 1, 3, 6, and 9, but are not limited thereto.

Additionally, the triple agonist may be one which has an increased *in vivo* half-life compared to any one of native GLP-1, native GIP, and native glucagon, but is not particularly limited thereto. The triple agonist of the present disclosure is characterized in that it exhibits activities for all of GLP-1 receptor, GIP receptor, and glucagon receptor, in particular, has a relatively low activity for the glucagon receptor, and at the same time, can maintain drug efficacy for a longer period of time due to the increased *in vivo* half-life, and thus can be used as an effective therapeutic agent for various diseases, such as obesity.

Although not particularly limited, the triple agonist may be one which is non-naturally occurring.

In one specific embodiment, the triple agonist may be a peptide including the amino acid sequence represented by General Formula 1 below:

X1-Aib-X3-G-T-F-T-S-D-Y-S-X12-X13-L-D-E-X17-X18-A-K-X21-F-V-Q-W-L-L-D-X29-H-P-S-S-G-Q-P-P-P-S (General Formula 1, SEQ ID NO: 29)

In General Formula 1 above,
X1 is histidine or tyrosine;
X3 is glutamine or histidine;
X12 is lysine or an acylated amino acid;
X13 is α-methyl-leucine, tyrosine, or alanine;
X17 is lysine or an acylated amino acid;
X18 is alanine or arginine;
X21 is aspartic acid or glutamic acid; and
X29 is histidine or glutamine;
wherein - represents a peptide bond, and the peptide does not contain cysteine.

Examples of the triple agonist according to the present disclosure may include those including the amino acid sequence of any one of SEQ ID NOS: 1 to 28, and those consisting (essentially) of the amino acid sequence of any one of SEQ ID NOS: 1 to 28, but are limited thereto.

More specifically, the triple agonist may be a peptide, wherein, in General Formula 1 above, X13 is tyrosine; and X18 is alanine, but is not limited thereto.

Examples of the triple agonist may include those including the amino acid sequence of any one of SEQ ID NOS: 1, 3, 6, and 9, and those consisting (essentially) of the amino acid sequence of any one of SEQ ID NOS: 1, 3, 6, and 9, but are not limited thereto.

Additionally, the triple agonist may be a peptide, wherein, in General Formula 1 above,
X1 is tyrosine;
X3 is glutamine;
X13 is tyrosine; and
X18 is arginine, but is not limited thereto.

Examples of the triple agonist may include those including the amino acid sequence of any one of SEQ **ID** NOS: 2, 4, 7, and 8, and those consisting (essentially) of the amino acid sequence of any one of SEQ **ID** NOS: 2, 4, 7, and 8, but are not limited thereto.

Additionally, the triple agonist may be a peptide, wherein, in General Formula 1 above, X29 is glutamine, but is not limited thereto.

Examples of the triple agonist may include those including the amino acid sequence of any one of SEQ **ID** NOS: 3, 5, 6, and 7, and those consisting (essentially) of the amino acid sequence of any one of SEQ **ID** NOS: 3, 5, 6, and 7, but are not limited thereto.

Additionally, the triple agonist may be a peptide, wherein, in General Formula 1 above, X3 is histidine, but is not limited thereto.

Examples of the triple agonist may include those including the amino acid sequence of SEQ ID NO: 6, and those consisting (essentially) of the amino acid sequence of SEQ ID NO: 6, but are not limited thereto.

In another specific embodiment, the triple agonist may be a peptide including the amino acid sequence represented by General Formula 2 below:

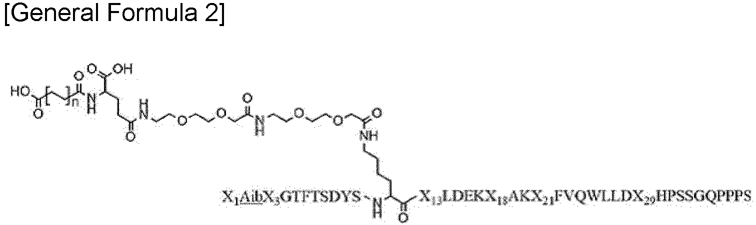

In General Formula 2 above,
n is 16 to 20, specifically, 16 or 18;
X1 is histidine or tyrosine;
X3 is glutamine or histidine;
X13 is α-methyl-leucine, tyrosine, or alanine;
X18 is alanine or arginine;
X21 is aspartic acid or glutamic acid; and
X29 is histidine or glutamine;
wherein, the peptide including the amino acid sequence represented by General Formula 2 above may not contain cysteine.

The peptide region of General Formula 2 above may be the same as the peptide of General Formula 1, and the technical contents with respect to General Formula 1 may also be applied to General Formula 2.

In one example of the triple agonist of General Formula 2, glutamic acid and lysine, which are the amino acids at position 16 and 20 respectively, may form a ring, specifically a lactam ring, but the triple agonist is not particularly limited thereto.

In the present disclosure, the triple agonist may be an acylated peptide, but is not limited thereto. In the present disclosure, the triple agonist may refer to both acylated or non-acylated triple agonists.

Acylation is known as a method for improving the pharmacokinetic and pharmacodynamic properties of peptide drugs. In the case of peptide drugs, there is a problem in that it is difficult to exert drug efficacy due to enzymatic degradation in the body. Accordingly, the stability and half-life of a peptide drug may be increased by blocking an enzyme active site through acylation of a peptide that attaches a fatty acid to the peptide. For the purpose of the present disclosure, the triple agonist of the present disclosure may be in an acylated form to increase half-life. The acylated triple agonist of the present disclosure has activities for all of the GLP-1 receptor, GIP receptor, and glucagon receptor and at the same time, has an increased *in vivo* half-life, and thus can be used as an effective therapeutic agent.

**In** the present disclosure, although it is described as "triple agonist" or "peptide" without a description of whether or not it is acylated, the acylated form thereof is not excluded, and may be used interchangeably with "acylated triple agonist" or "acylated peptide".

**The** acylated triple agonist of the present disclosure may have an acyl group directly linked to the amino acid of the triple agonist peptide, or may have an acyl group attached through a linker. Although not limited, the linker may be a linker selected from a group consisting of (2-(2-aminoethoxy)ethoxy)acetic acid (AEEA), 4-Aminobutyric acid (GABA), 5-Aminovaleric acid (Ava), Aminohexanoic acid (Ahx), triazole, and polyethylene glycol (PEG).

**In** one specific example, the linker may include (2-(2-aminoethoxy)ethoxy)acetic acid (AEEA), and more specifically, gamma-glutamate may be additionally linked, but is not limited thereto. Specific examples of the linker may include (gammaGlu)ₘ-(AEEA)ₙ, where m and n may each independently be integers of 0, 1, 2, 3, or higher, but are not limited thereto.

**In** another example, the linker may be polyethylene glycol (maleimide-PEG) having a maleimide reactive group, but is not limited thereto.

**In** still another example, the triple agonist peptide and the acyl group may be linked through a click chemical reaction, but is not limited thereto.

**The** acyl group may be bonded to the amine group, hydroxy group, thiol group, carboxyl group, *etc.* of the amino acid of the triple agonist through the amine group, hydroxy group, thiol group, *etc.* of the linker, but is not limited to a particular type or length as long as it can contribute to increasing the structural stability and half-life of the triple agonist by binding the acyl group to the triple agonist. Additionally, the linker may be covalently bonded to the acyl group, and may be linked to the acyl group by repeating 1, 2, 3, or more times, but is not limited thereto.

In the present disclosure, the acyl group may be a carbon chain having an arbitrary length, and may be linear or branched. Specifically, the chain may include a linear aliphatic chain, a branched aliphatic chain, a chain containing a cyclic alkyl moiety, a hydrophobic natural product such as a steroid, an aralkyl chain, or an alkyl chain containing an acyl moiety.

Although not limited, the acylated triple agonist may be acylated with a C₁ to C₃₀ straight-chain or branched-chain acyl group containing one or more, two or more, specifically one or two carboxylic acids. For example, the acyl group may be a fatty acid or dicarboxylic acid, specifically, a C₄ to C₄₀ fatty acid or dicarboxylic acid, but is not limited thereto. More specifically, it may be a C₁₆, C₁₈, C₂₀, C₂₂, C₂₄, C₂₆, C₂₈, or C₃₀ fatty acid or dicarboxylic acid. In another example, the acyl group may include cholic acid, chenodeoxycholic acid, deoxycholic acid, lithocholic acid, taurocholic acid, glycocholic acid, bile acid such as cholesterol acid, succinic acid or succinic acid derivatives, or maleic acid or maleic acid derivatives, but is not limited thereto.

In one specific embodiment, the acyl group may be a C₁₈ or higher fatty acid, a fatty diacid, or derivatives thereof, more specifically, octadecanoic acid, octadecanedioic acid, linoleic acid, linolenic acid. oleic acid, *etc.,* but is not limited thereto.

In another specific embodiment, the acyl group may be a C₂₀ or higher fatty acid, fatty diacid, or derivatives thereof, more specifically, eicosanoic acid, docosanoic acid, eicosapentaenoic acid, adrenic acid, *etc.,* but is not limited thereto.

The acylated triple agonist of the present disclosure may be one in which an acyl group is attached to the triple agonist by a method known in the art, or may be prepared by synthesizing a peptide using an acylated amino acid, but is not limited thereto.

In the present disclosure, the acylated triple agonist may be in a form in which an acyl group is attached directly to an amino acid residue of the triple agonist. For example, an acyl group may be attached through an ester, thioester, imide, or amide bond, but is not limited thereto. Specifically, the acylated triple agonist may be acylated at an amino acid residue having an amine, hydroxyl, or thiol group. For example, the acylated triple agonist may be acylated at an amino acid located at the N-terminus or C-terminus or lysine residue, but is not limited thereto.

In the present disclosure, the acylated triple agonist or the acylated amino acid of General Formula 1 may include any one among the amino acid represented by K(1) or K(2) below, but is not limited thereto:
[K(1)] C20diacid-gammaGlu-(AEEA)₂-Lys 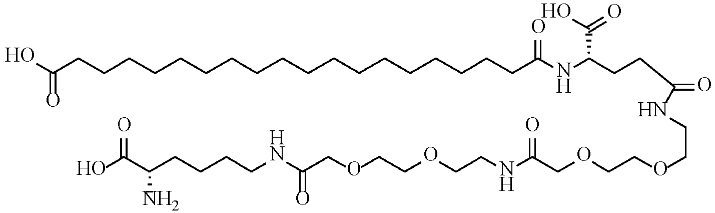
[K(2)] C18diacid-gammaGlu-(AEEA)₂-Lys

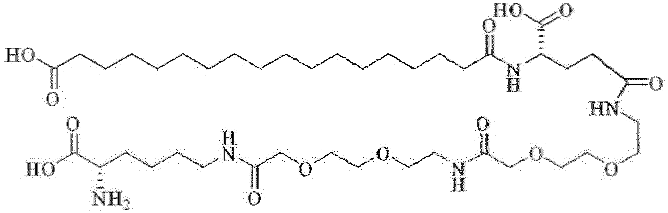

The acylated amino acid may be linked to another amino acid in the peptide. Specifically, the lysine of K(1) and K(2) may be linked to another amino acid adjacent to the triple agonist through a carboxyl group and/or amino group, and may be linked to a linker and an acyl group through a side chain. It is apparent that forms linked to other amino acids are included in the scope of K(1) and K(2) of the present disclosure.

The triple agonist of the present disclosure may be acylated at one or more amino acids at positions 1 to 39 from the N-terminus, or at the N-terminus or C-terminus, but the location of acylation or the number of acylated amino acids is not limited as long as it has activity as a triple agonist.

In one specific example, the acylated triple agonist of the present disclosure may include an acylated amino acid at any one or more of positions 12, 17, and 20, but is not limited thereto.

In one specific embodiment of the acylated triple agonist of the present disclosure, it may include an acylated amino acid at position 12. Specifically, it may include K(1) at position 12, more specifically, the amino acid sequence of any one of SEQ ID NOS: 1 to 7, or it may include K(2) at position 12, more specifically, the amino acid sequence of any one of SEQ ID NOS: 8 to 14, but is not limited thereto.

In another specific embodiment of the acylated triple agonist of the present disclosure, it may include an acylated amino acid at position 17. Specifically, it may include K(1) at position 17, more specifically, the amino acid sequence of any one of SEQ ID NOS: 15 to 21, or it may include K(2) at position 17, more specifically, the amino acid sequence of any one of SEQ ID NOS: 22 to 28, but is not limited thereto.

In still another specific embodiment, the triple agonist may include the amino acid sequence of any one of SEQ ID NOS: 1 to 28, or may consist (essentially) of the amino acid sequence of any one of SEQ ID NOS: 1 to 28, but is not limited thereto.

In the present disclosure, although it is described as 'a peptide consisting of a particular SEQ ID NO:', such description does not exclude an addition of a meaningless sequence upstream or downstream of the amino acid sequence of the corresponding SEQ ID NO, or a mutation that may occur naturally, or a silent mutation thereof, as long as the peptide has an activity identical or corresponding to that of the peptide which consists of the amino acid sequence of the corresponding SEQ ID NO, and it obviously belongs to the scope of the present disclosure even when the peptide has such a sequence addition or mutation therein.

Additionally, the triple agonist of the present disclosure may include an amino acid sequence having 40%, 45%, 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% homology or identity to the amino acid sequence of SEQ ID NOS: 1 to 28, but is not limited thereto as long as it exhibits activity by acting on the GLP-1 receptor, GIP receptor, and glucagon receptor.

As used herein, the term 'homology' or 'identity' refers to the degree of relatedness between two given amino acid sequences or nucleotide sequences, and may be expressed as a percentage.

The sequence homology or identity of conserved peptides may be determined by standard alignment algorithms, and may be used together with a default gap penalty established by the program being used. Substantially, homologous or identical sequences are generally expected to hybridize to all or part of the sequences under moderate or high stringent conditions. It is apparent that hybridization also includes hybridization with polynucleotides containing general codons or codons in consideration of codon degeneracy in polynucleotides.

The terms homology and identity may be often used interchangeably with each other.

Whether any two peptide sequences have homology, similarity, or identity may be determined by a known computer algorithm such as the "FASTA" program using default parameters as in Pearson et al. (1988) Proc. Natl. Acad. Sci. USA 85:2444. Alternatively, it may be determined by the Needleman-Wunsch algorithm (Needleman and Wunsch, 1970, J. Mol. Biol. 48:443-453), which is performed using the Needleman program of the EMBOSS package (EMBOSS: The European Molecular Biology Open Software Suite, Rice et al., 2000, Trends Genet. 16:276-277) (version 5.0.0 or later) (GCG program package (Devereux, J. et at, Nucleic Acids Research 12:387 (1984)), BLASTP, BLASTN, FASTA (Atschul, S. F. et al., J MOLEC BIOL 215:403 (1990); Guide to Huge Computers, Martin J. Bishop, ed., Academic Press, San Diego, 1994, and CARILLO et al. (1988) SIAM J Applied Math 48:1073). For example, homology, similarity, or identity may be determined using BLAST or ClustalW of the National Center for Biotechnology Information.

The homology, similarity, or identity of peptides may be determined by comparing sequence information using, for example, the GAP computer program, such as Needleman et al. (1970), J Mol Biol. 48:443, as disclosed in Smith and Waterman, Adv. Appl. Math (1981) 2:482. In summary, the GAP program defines the homology, similarity or identity as the value obtained by dividing the number of similarly aligned symbols (i.e., nucleotides or amino acids) by the total number of the symbols in the shorter of the two sequences. Default parameters for the GAP program may include (1) a binary comparison matrix (containing a value of 1 for identities and 0 for non-identities) and the weighted comparison matrix (or EDNAFULL (EMBOSS version of NCBI NUC4.4) substitution matrix) of Gribskov et al. (1986) Nucl Acids Res. 14:6745, as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or a gap opening penalty of 10 and a gap extension penalty of 0.5); and (3) no penalty for end gaps. Accordingly, as used herein, the term "homology" or "identity" refers to the relevance between sequences.

In one specific example of the triple agonist of the present disclosure, the triple agonist may be a peptide having any one structure from (i) to (iv) below, but is not limited thereto:

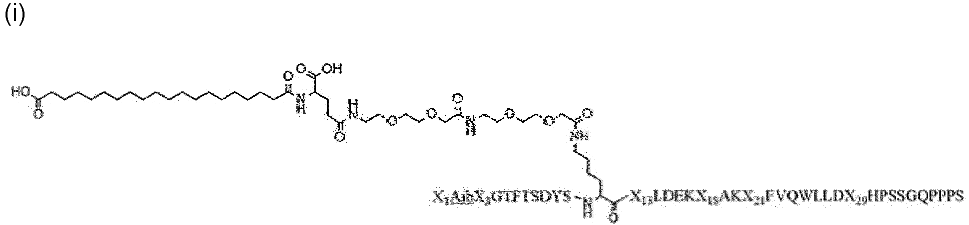

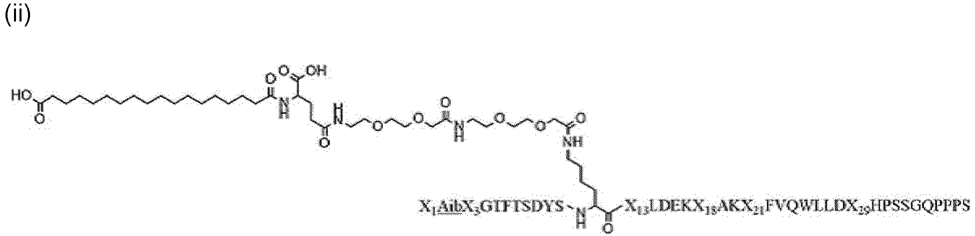

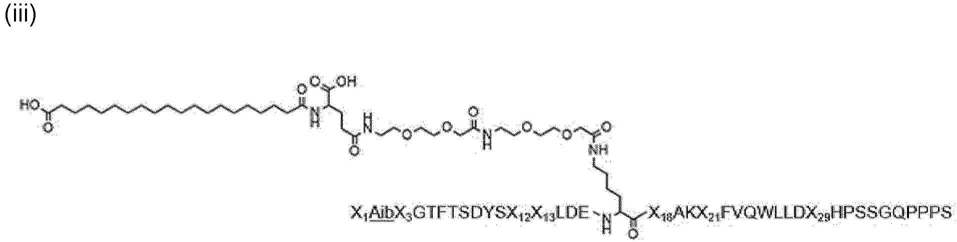

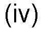

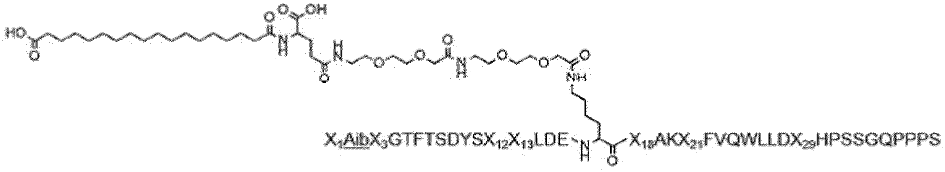

Meanwhile, the triple agonist according to the present disclosure does not contain cysteine (or derivatives thereof) within or at both ends of the peptide, and may consist of natural or non-natural amino acid residues excluding cysteine.

Additionally, the triple agonist of the present disclosure may be one in which the N-terminus and/or C-terminus of the peptide is not modified, however, those variants, where the N-terminus and/or C-terminus, *etc.,* of the peptide is chemically modified or protected by organic groups, or amino acids are added to the end of the peptide, *etc.,* for its protection from proteases *in vivo* while increasing its stability, may also be included in the scope of the present disclosure.

In particular, in the case of a chemically-synthesized peptide, its N- and C-termini are electrically charged, and thus the N-terminus may be acetylated and/or the C-terminus may be amidated, but the peptide is not particularly limited thereto.

The triple agonist according to the present disclosure may have an unmodified C-terminus or may have an amidated C-terminus, but is not limited thereto.

Meanwhile, the triple agonist of the present disclosure may include an intramolecular bridge (*e.g*., a covalent bridge or a non-covalent bridge), and specifically, may be in a form including a ring. For example, the triple agonist may be in a form where a ring is formed between the amino acids at positions 16 and 20 and/or the amino acids at positions 17 and 21 of the triple agonist, but is not particularly limited thereto.

Non-limiting examples of the ring may include a lactam bridge (or lactam ring).

The triple agonist of the present disclosure includes all of those which are modified to include an amino acid capable of forming a ring at the desired site so as to include a ring.

For example, the triple agonist of the present disclosure may have glutamic acid or lysine, which are capable of forming a ring between the amino acid pair at positions 16 and 20 and/or the amino acid pair at positions 17 and 21, but is not limited thereto.

Such a ring may be formed between side chains of amino acids within the triple agonist, e.g., the ring may be in a form where a lactam ring is formed between a side chain of lysine and a side chain of a glutamic acid), but is not particularly limited thereto.

Additionally, although not particularly limited, some of the amino acids may be substituted with other amino acids or non-natural compounds to avoid recognition by a degradation enzyme of the triple agonist for increasing the *in vivo* half-life.

Specifically, the triple agonist may be a peptide in which the *in vivo* half-life is increased by avoiding recognition by the degradation enzyme through a substitution of the 2^{nd} amino acid sequence among the amino acid sequences of the triple agonist, but any substitution or modification of amino acids to avoid recognition by an *in vivo* degradation enzyme is included without limitation.

Additionally, the triple agonist of the present disclosure may include all of the modifications using L-type or D-type amino acids, and/or non-native type amino acids; and/or a modification of native sequence, for example, modification of a functional group, an intramolecular covalent bonding (*e.g*., a ring formation between side chains), methylation, acylation, ubiquitination, phosphorylation, aminohexanation, biotinylation, *etc.*

Additionally, the modification also includes all of those where one or more amino acids are added to the amino and/or carboxy terminus of the triple agonist of the present disclosure.

As the amino acids for the substitution or addition, not only the 20 amino acids commonly found in human proteins, but also atypical or non-naturally occurring amino acids may be used. Commercial sources of atypical amino acids include Sigma-Aldrich, ChemPep Inc., and Genzyme Pharmaceuticals. The peptides, where these amino acids are included, and typical peptide sequences may be synthesized and purchased from commercial peptide synthesis companies (*e.g*., American Peptide Company, Bachem (USA), or Anygen (Korea)).

Amino acid derivatives may be obtained in the same manner, for example, 4-imidazoacetic acid or α-methyl-leucine (αMeL), *etc.* may be used.

Unless specified otherwise in the present disclosure, the description in the detailed description or claims with respect to the triple agonist according to the present disclosure may be applied to the forms, which include not only include the corresponding triple agonist, but also salts of the corresponding peptide (triple agonist) (*e.g*., pharmaceutically acceptable salts thereof), or solvates thereof. Accordingly, the description of the triple agonist throughout the specification may also be equally applied to a particular salt thereof, a particular solvate thereof, and a particular solvate of the particular salt thereof. These salts may be, for example, in a form where any pharmaceutically acceptable salts are used. The kind of the salt is not particularly limited. However, the salt is preferably one that is safe and effective to a subject, *e.g*., a mammal, but is not particularly limited thereto.

The term "pharmaceutically acceptable" refers to a material which can be effectively used for the intended use within the scope of pharmaco-medical decision without inducing excessive toxicity, irritation, allergic responses, *etc.*

As used herein, the term "pharmaceutically acceptable salt" refers to a salt derived from pharmaceutically acceptable inorganic salts, organic salts, or bases. Examples of the suitable salts may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, benzenesulfonic acid, *etc.* Examples of the salts derived from suitable bases may include alkali metals such as sodium, potassium, *etc.;* alkali earth metals such as magnesium; ammonium, *etc.*

Additionally, as used herein, the term "solvate" refers to a complex formed between the triple agonist according to the present disclosure or a salt thereof, and a solvent molecule.

Additionally, the triple agonist of the present disclosure may be synthesized according to its length by a method well known in the art (e.g., by an automatic peptide synthesizer), and may be also produced by genetic engineering technology.

Specifically, the triple agonist of the present disclosure may be prepared by a standard synthesis method, a recombinant expression system, or any other method known in the art. Accordingly, the triple agonist of the present disclosure may be synthesized by various methods including, for example, the methods described below:
(a) a method of synthesizing a peptide by a solid-phase or liquid-phase method stepwise or by fragment assembly, followed by isolation and purification of the final peptide product; or
(b) a method of expressing a nucleic acid construct encoding a peptide in a host cell and recovering the expression product from the host cell culture;
(c) a method of performing an *in vitro* cell-free expression of a nucleic acid construct encoding a peptide and recovering the expression product therefrom; or
a method of obtaining peptide fragments by any combination of the methods (a), (b), and (c), obtaining the peptide by linking the peptide fragments, and then recovering the peptide.

Those described above may be also applied to other embodiments or aspects of the present disclosure, but is not limited thereto.

Another aspect for implementing the present disclosure provides a polynucleotide encoding the triple agonist, a recombinant expression vector containing the polynucleotide, and a transformant containing the polynucleotide or recombinant expression vector.

The triple agonist is as described above.

Additionally, the isolated polynucleotide encoding the triple agonist may include polynucleotide sequences having a sequence identity of 75% or higher, specifically 85% or higher, more specifically 90% or higher, and even more specifically 95% or higher with the corresponding sequence, and those are included in the scope of the present disclosure.

As used herein, the term "homology" is intended to indicate sequence similarity with a wild-type amino acid sequence or wild-type nucleotide sequence, and the homology comparison may be performed with the naked eye or using a commercially available comparison program. Using a commercially available computer program, the homology between two or more sequences may be expressed as a percentage (%), and the homology (%) between adjacent sequences may be calculated.

As used herein, the term "recombinant vector" refers to a DNA construct in which a target peptide is operably linked to an appropriate regulatory sequence to enable the expression of the target peptide in an appropriate host. The vector may be an expression vector for expressing the polynucleotide in the host cell, but is not limited thereto.

The vector of the present disclosure may include a DNA construct including the nucleotide sequence of a polynucleotide encoding a target peptide, which is operably linked to an appropriate expression regulatory region (or expression regulatory sequence) to enable the expression of the target peptide in an appropriate host.

The regulatory sequence includes a promoter capable of initiating transcription, any operator sequence for regulating such transcription, a sequence encoding a suitable mRNA ribosome binding site, and a sequence for regulating the termination of transcription and translation. The recombinant vector may be transformed into an appropriate host cell, and then may replicate or function regardless of a host genome or may be integrated into the genome itself.

The recombinant vector used in the present disclosure is not particularly limited, as long as it is replicable in a host cell, and may be prepared using any vector known in the art. Examples of generally-used vectors may include native or recombinant plasmids, cosmids, viruses, and bacteriophages. The vector applicable in the present disclosure is not particularly limited, and any known expression vector may be used.

In order to produce the triple agonist of the present disclosure, the recombinant vector is used to transform host cells. In addition, the transformed cell, which are part of the present disclosure, may be a cultured cell or cell line used for propagation of the nucleic acid fragment and the vector of the present disclosure, or for recombinant production of the triple agonist of the present disclosure.

As used herein, the "transformation" means introduction of a recombinant vector including a polynucleotide encoding a target protein into a host cell in such a way that the protein encoded by the polynucleotide is expressed in the host cell. As long as the transformed polynucleotide may be expressed in the host cell, it may be inserted into and located in the chromosome of the host cell or exist extrachromosomally, or it may be in any of the cases.

Further, the polynucleotide includes DNA and RNA encoding the target protein. The polynucleotide may be introduced in any form, as long as it may be introduced into the host cell and expressed therein. For example, the polynucleotide may be introduced into the host cell in the form of an expression cassette, which is a gene construct including all elements required for its autonomous expression. Commonly, the expression cassette may include a promoter operably linked to the polynucleotide, transcriptional termination signals, ribosome binding sites, and translation termination signals. The expression cassette may be in the form of a self-replicable expression vector. Additionally, the polynucleotide as it is may be introduced into the host cell and operably linked to sequences required for expression in the host cell, but is not limited thereto.

As used herein, the term "operably linked" means a functional linkage between a promoter sequence, which initiates and mediates transcription of the polynucleotide encoding the target peptide in the present disclosure, and the gene sequence.

The host suitable for the present disclosure is not particularly limited, as long as it enables the expression of the polynucleotide of the present disclosure. Specific examples of the host applicable in the present disclosure may include bacteria of the genus *Escherichia* such as *E. coli;* bacteria of the genus *Bacillus* such as *Bacillus subtilis;* bacteria of the genus *Pseudomonas* such as *Pseudomonas putida;* yeasts such as *Pichia pastoris, Saccharomyces cerevisiae,* or *Schizosaccharomyces pombe;* insect cells such as *Spodoptera frugiperda* (Sf9); and animal cells such as CHO, COS, BSC, *etc.*

Still another aspect for implementing the present disclosure provides a method for preparing the triple agonist.

The triple agonist is as described above.

Additionally, the triple agonist of the present disclosure may be synthesized according to its length by a method well known in the art (e.g., by an automatic peptide synthesizer), and may also be produced by genetic engineering technology.

Specifically, the triple agonist of the present disclosure may be prepared by a standard synthesis method, a recombinant expression system, or any other method known in the art. Accordingly, the triple agonist of the present disclosure may be synthesized by various methods including, for example, the methods described below:
(a) a method of synthesizing a peptide by a solid-phase or liquid-phase method stepwise or by fragment assembly, followed by isolation and purification of the final peptide product; or
(b) a method of expressing a nucleic acid construct encoding a peptide in a host cell and recovering the expression product from the host cell culture;
(c) a method of performing an *in vitro* cell-free expression of a nucleic acid construct encoding a peptide and recovering the expression product therefrom; or
a method of obtaining peptide fragments by any combination of the methods (a), (b), and (c), obtaining the peptide by linking the peptide fragments, and then recovering the peptide.

In more specific example, a desired peptide may be produced, through genetic manipulation, by preparing a fusion gene encoding a fusion protein including a fusion partner and the triple agonist, transforming the fusion gene into a host cell, expressing the fusion gene in the form of a fusion protein, and cleaving and separating the peptide from the fusion protein using a protease or a compound. For this purpose, for example, an amino acid residue-encoding DNA sequence that may be cleaved by a protease such as Factor Xa or enterokinase, or a compound such as CNBr or hydroxylamine, may be inserted between the fusion partner and a polynucleotide encoding the peptide.

Yet another aspect for implementing the present disclosure provides a composition including the triple agonist.

The triple agonist is as described above.

Specifically, the composition may be a pharmaceutical composition, and more specifically, the composition may be used for preventing or treating obesity.

One specific aspect of the present disclosure provides a pharmaceutical composition for treating or preventing obesity including the triple agonist in a pharmaceutically effective amount. The triple agonist included in the composition may also include an acylated form.

One example of the triple agonist included in the composition may include, but is not limited to, a peptide including any one of the amino acid sequences of SEQ ID NOS: 1 to 28.

Another example of the triple agonist included in the composition may include, but is not limited to, a peptide having any one of structures from (i) to (iv) below:

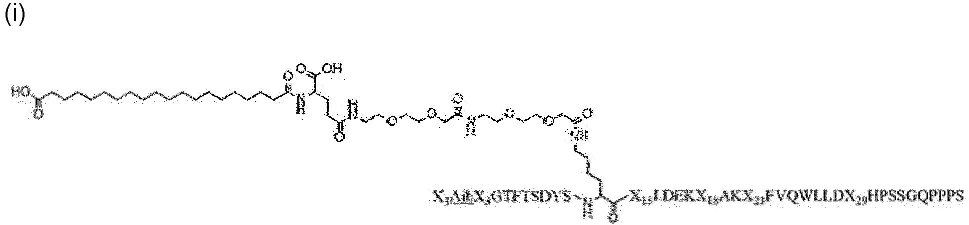

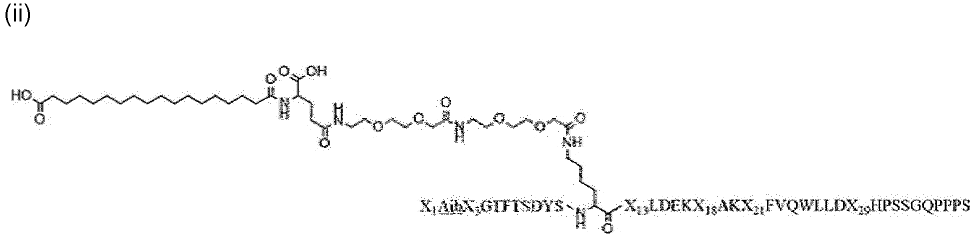

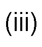

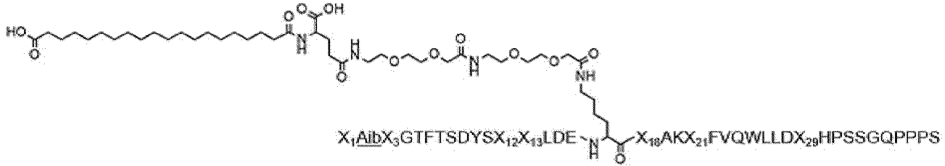

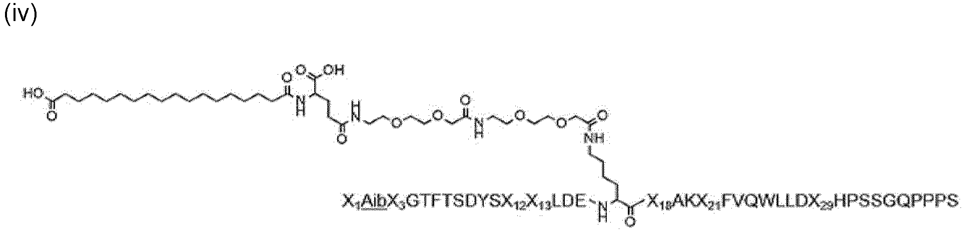

Still another example of the triple agonist included in the composition may include, but is not limited to, a peptide having the structure of General Formula 2 below:

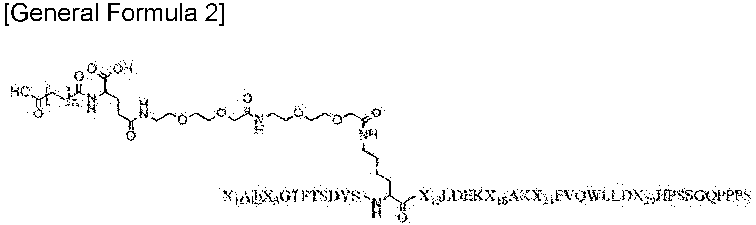

In General Formula 2 above,
n is 16 or 18;
X1 is histidine or tyrosine;
X3 is glutamine or histidine;
X13 is α-methyl-leucine, tyrosine, or alanine;
X18 is alanine or arginine;
X21 is aspartic acid or glutamic acid; and
X29 is histidine or glutamine.

The peptide having the structure of General Formula 2 is as described above, and all descriptions of the peptide having the structure of Formula 1 maybe be applied.

Containing the triple agonist in a pharmaceutically effective amount refers to a level at which the desired pharmacological activity (e.g., prevention, improvement, or treatment of obesity) may be obtained, and in addition, may refer to a pharmaceutically acceptable level, which is a level at which toxicities or adverse effects do not occur or occur at an insignificant level in the subject to be administered, but the level is not limited thereto. The pharmaceutically effective amount may be determined by comprehensively considering the number of administration, patient, formulations, *etc.*

As used herein, the term "prevention" refers to all activities that inhibit or delay the occurrence of a target disease (e.g., obesity) by administering the triple agonist (including the acylated form), or a composition including the same, and the term "treatment" refers to all activities that improve or advantageously change the symptoms of a target disease (*e.g*., obesity) by administering the triple agonist (including the acylated form), or a composition including the same.

As used herein, the term "administration" refers to the introduction of a particular material into a patient by any appropriate method, and the administration route of the composition is not particularly limited, but may be any conventional route that enables delivery of the composition to the target *in vivo* (*e.g.,* intraperitoneal administration, intravenous administration, intramuscular administration, subcutaneous administration, intradermal administration, oral administration, topical administration, intranasal administration, intrapulmonary administration, intrarectal administration, *etc*.).

As used herein, the term "obesity" refers to excessive accumulation of adipose tissue in the body, and is defined as a body mass index (weight (kg) divided by height (m) squared) above 25. Obesity is usually caused by long-term energy imbalance between an excessive intake of nutrients and energy consumption. Obesity is a metabolic disease that affects the entire body, and increases the risk of diabetes and hyperlipidemia, and the risk of sexual dysfunction, arthritis, cardiovascular disease, and in some cases, is associated with cancer.

The triple conjugate of the present disclosure may exhibit a weight loss effect by acting on the GLP-1 receptor, GIP receptor, and glucagon receptor, and in particular, it has higher relative activities for the GLP-1 receptor and GIP receptor relative to native GLP-1 and GIP, respectively, compared to the relative activity for the glucagon receptor relative to native glucagon, thereby exhibiting a preventing or therapeutic effect on obesity, but is not limited thereto.

The DIO diet and AMLN diet-induced models used in the Examples of the present disclosure are known as obesity models. The above models are a model used in obesity-related research, and in the Examples of the present disclosure, it was confirmed that the triple agonist according to the present disclosure exhibited an excellent weight loss effect in all of the above models, which implies that the triple agonist according to the present disclosure is effective in preventing or treating obesity.

The pharmaceutical composition of the present disclosure may further include a pharmaceutically acceptable carrier, excipient, or diluent. As used herein, the term "pharmaceutically acceptable" refers to the properties of having a sufficient amount to exhibit a therapeutic effect and not causing adverse effects, and may be easily determined by a skilled person in the art based on the factors well known in the medical field, such as the kind of disease, age, body weight, health status, sex, drug sensitivity of a patient, administration route, administration method, administration frequency, duration of treatment, a drug to be mixed or administered simultaneously in combination, *etc.*

The pharmaceutical composition of the present disclosure including the triple agonist of the present disclosure may further include a pharmaceutically acceptable carrier. The pharmaceutically acceptable carrier may include, for oral administration, a binder, a lubricant, a disintegrant, a solubilizing agent, a dispersant, a stabilizing agent, a suspending agent, a coloring agent, a fragrance, *etc.;* for injections, a buffering agent, a preservative, an analgesic, a solubilizing agent, an isotonic agent, a stabilizing agent, *etc.,* which may be combined to be used; and for topical administrations, a base, an excipient, a lubricant, a preservative, *etc.,* although it is not particularly limited thereto.

The formulation type of the composition according to the present disclosure may be prepared variously in combination with a pharmaceutically acceptable carrier described above. For example, for oral administration, the composition may be formulated into tablets, troches, capsules, elixirs, suspensions, syrups, wafers, *etc.,* and for injections, the composition may be formulated into unit-dose ampoules or multi-dose containers. The composition may also be formulated into solutions, suspensions, tablets, pills, capsules, sustained-release formulations, *etc.*

Meanwhile, examples of suitable carriers, excipients, and diluents for formulation may include lactose, dextrose, sucrose, sorbitol, mannitol, xylitol, erythritol, maltitol, starch, acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, or mineral oil, *etc.* Additionally, the composition may further include a filler, an anticoagulant, a lubricant, a humectant, a fragrance, a preservative, *etc.*

Additionally, the pharmaceutical composition of the present disclosure may have any one formulation type selected from the group consisting of tablets, pills, powders, granules, capsules, suspensions, liquid medicine for internal use, emulsions, syrups, sterile aqueous solutions, non-aqueous solvents, lyophilized formulations, and suppositories.

Additionally, the composition may be formulated in a unit dosage form suitable for administration into a patient's body, specifically in a form useful for administration of peptide drugs according to the conventional method in the pharmaceutical field so as to be administered via an oral administration route or a parenteral administration route such as an intradermal, intravenous, intramuscular, intraarterial, intramedullary, intrathecal, intraventricular, intrapulmonary, transdermal, subcutaneous, intraperitoneal, intranasal, intestinal, topical, sublingual, vaginal, or rectal route using an administration method commonly used in the art, but is not limited thereto.

Additionally, the triple agonist may be used in combination with various carriers permitted as medicaments, such as physiological saline or organic solvents. To increase stability or absorptivity, carbohydrates (*e.g*., glucose, sucrose, or dextran), antioxidants (*e.g*., ascorbic acid or glutathione), chelating agents, low-molecular weight proteins, or other stabilizers, *etc.* may be used as medicaments.

The administration dose and frequency of the pharmaceutical composition of the present disclosure are determined by the type of drugs, which are active ingredients, together with various factors, such as the disease to be treated, administration route, patient's age, sex, and body weight, severity of the disease, *etc.*

Although not particularly limited thereto, the pharmaceutical composition of the present disclosure may contain the above ingredients (active ingredients) in an amount of 0.01% to 99% (W/V).

The total effective dose of the composition of the present disclosure may be administered to a patient in a single dose or may be administered for a long period of time in multiple doses according to a fractionated treatment protocol. In the pharmaceutical composition of the present disclosure, the content of active ingredient(s) may vary depending on the severity of the disease. Specifically, the administration cycle of the triple agonist of the present disclosure may be one day to one week, and the dose administered per week may be about 0.01 to 0.9 mg per 1 kg of the body weight of a patient, but is not limited thereto. In the case of an acylated triple agonist, the dose may be determined based on the mass value excluding the mass of the fatty acid portion of the triple agonist, *i.e*., the sum of the masses of only the polypeptide portions. However, the effective dose of the triple agonist is determined considering various factors including patient's age, body weight, health conditions, sex, severity of disease, diet, and excretion rate, as well as administration route and treatment frequency of the pharmaceutical composition. In this respect, those skilled in the art may easily determine the effective dose suitable for a particular use of the pharmaceutical composition of the present disclosure. The pharmaceutical composition according to the present disclosure is not particularly limited to the formulation type, and administration route and method, as long as it shows the effects of the present disclosure.

The pharmaceutical composition of the present disclosure may have excellent *in vivo* duration and titer, thereby reducing the number and frequency of administration compared to other medicaments, but is not particularly limited thereto.

Even another aspect for implementing the present disclosure provides a method for preventing or treating obesity, including administering the triple agonist or a composition including the same to a subject.

The triple agonist, composition including the same, obesity, prevention, and treatment are as described above.

As used herein, the subject refers to a subject suspected of having obesity, and the subject suspected of having obesity refers to mammals including humans, rats, livestock, *etc.,* which have or are at risk of developing obesity, but any subject which can be treated with the triple agonist of the present disclosure or the composition including the same is included without limitation. Additionally, by administering the pharmaceutical composition including the triple agonist of the present disclosure to a subject suspected of having obesity, the subject can be efficiently treated. The obesity is as described above.

The method of the present disclosure may include administering the pharmaceutical composition including the triple agonist in a pharmaceutically effective amount. An appropriate total daily dose of the pharmaceutical composition may be determined within the scope of correct medical judgment by a practitioner, and the pharmaceutical composition may be administered once or several times in divided doses. However, for the purpose of the present disclosure, it is preferred that the specific therapeutically effective dose of the pharmaceutical composition for any particular patient be applied differently depending on the kind and degree of responses to be achieved, specific compositions including whether other agents are occasionally used therewith, the patient's age, body weight, health conditions, sex and diet, administration time, administration route, excretion rate of the composition, duration of treatment, various factors including drugs used in combination or simultaneously with the specific compositions, and similar factors well known in the medical field.

In the method of the present disclosure, the triple agonist or a composition including the same may be administered through a conventional route that can reach the target *in vivo,* and the administration route includes, for example, intraperitoneal administration, intravenous administration, intramuscular administration, subcutaneous administration, intradermal administration, oral administration, topical administration, intranasal administration, intrapulmonary administration, intrarectal administration, *etc.*

Further another aspect for implementing the present disclosure provides the use of the triple agonist or composition for the prevention or treatment of obesity.

The triple agonist, composition, obesity, prevention and treatment are as described above.

Still further another aspect for implementing the present disclosure provides the use of the triple agonist or composition in the preparation of a medicament (or pharmaceutical composition) for the prevention or treatment of obesity.

The triple agonist, composition, obesity, prevention and treatment are as described above.

Meanwhile, in the present specification, unless the context requires otherwise, the expressions "include", "including", "containing", *etc.* mean the inclusion of the specified integer or group of integers, but it should be understood that other integers or sets of integers are not excluded.

Hereinafter, the present disclosure will be described in more detail with reference to the following Examples. However, these Examples are for illustrative purposes only, and the scope of the present disclosure is not intended to be limited by these Examples.

### Example: Preparation of Triple Agonist

The triple agonist of the present disclosure was synthesized by an automatic peptide synthesizer (Symphony X, Gyros Protein Tech.) using a solid-phase synthesis method. Rink amide resin was used for amidation of the C-terminus, and each amino acid was synthesized in the order from the C-terminus to the N-terminus.

Amino acids were sequentially bonded using Fmoc (9H-Fluoren-9-ylmethoxycarbonyl) protected amino acid, (4 equivalents relative to peptide-resin), HOBt (1-hydroxybenzotriazole, 4 equivalents relative to peptide-resin), and DIC (diisopropylcarbodiimide, 8 equivalents relative to peptide-resin). The same method as above was used when binding acylated amino acids, K(1) or K(2).
[K(1)] C20diacid-gammaGlu-(AEEA)2-Lys 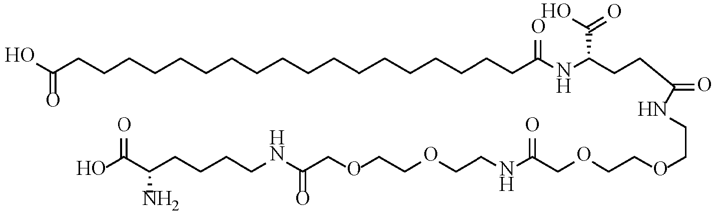
[K(2)] C18diacid-gammaGlu-(AEEA)2-Lys

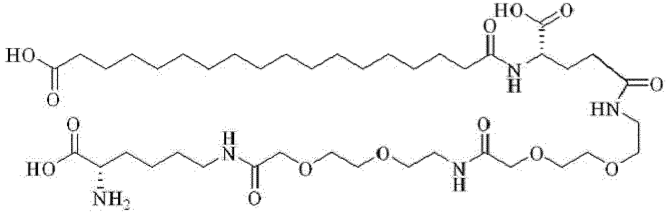

The Fmoc-protecting group was removed by adding 8 mL of 20% piperidine/DMF (2 x 5 minutes) to the reaction vessel containing the resin in the automatic synthesizer. To remove remaining impurities, washing was performed with 12 mL of DMF (6 x 10 seconds) after each process. Protecting groups that were not removed were also deprotected when the peptide was cleaved from the resin.

The sequences of the triple agonists prepared through the above process are shown in Table 1 below.

**[Table 1]**

| SEQ ID NO: | Amino acid sequences |
|---|---|
| 1 | Y-Aib-QGTFTSDYSK(1)YLDEKAAKEFVQWLLDHHPSSGQPPPS |
| 2 | Y-Aib-QGTFTSDYSK(1)YLDEKRAKEFVQWLLDHHPSSGQPPPS |
| 3 | Y-Aib-QGTFTSDYSK(1)YLDEKAAKEFVQWLLDHHPSSGQPPPS |
| 4 | Y-Aib-QGTFTSDYSK(1)YLDEKRAKEFVQWLLDHHPSSGQPPPS |
| 5 | Y-Aib-QGTFTSDYSK(1)ALDEKRAKEFVQWLLDQHPSSGQPPPS |
| 6 | H-Aib-QGTFTSDYSK(1)YLDEKAAKEFVQWLLDHHPSSGQPPPS |
| 7 | Y-Aib-QGTFTSDYSK(1)YLDEKRAKEFVQWLLDHHPSSGQPPPS |
| 8 | Y-Aib-QGTFTSDYSK(2)YLDEKRAKEFVQWLLDHHPSSGQPPPS |
| 9 | Y-Aib-QGTFTSDYSK(2)YLDEKAAKEFVQWLLDHHPSSGQPPPS |
| 10 | Y-Aib-QGTFTSDYSK(2)YLDEKAAKEFVQWLLDHHPSSGQPPPS |
| 11 | Y-Aib-QGTFTSDYSK(2)YLDEKRAKDFVQWLLDHHPSSGQPPPS |
| 12 | Y-Aib-QGTFTSDYSK(2)ALDEKRAKEFVQWLLDQHPSSQPPPS |
| 13 | H-Aib-HGTFTSDYSK(2)YLDEKAAKDFVQWLLDQHPSSGQPPPS |
| 14 | Y-Aib-QGTFTSDYSK(2)YLDEKRAKDFVQWLLDQHPSSGQPPPS |
| 16 | Y-Aib-QGTFTSDYSKYLDEK(1)AAKEFVQWLLDHHPSSGQPPPS |
| 16 | Y-Aib-QGTFTSDYSKYLDEK(1)RAKEFVQWLLDHHPSSGQPPPS |
| 17 | Y-Aib-QGTFTSDYSKYLDEK(1)AAKDFVQWLLDQHPSSGQPPPS |
| 18 | Y-Aib-QGTFTSDYSKYLDEK(1)RAKDFVQWLLDHHPSSGQPPPS |
| 19 | Y-Aib-QGTFTSDYSKALDEK(1)RAKEFVQWLLDQHPSSGQPPPS |
| 20 | H-Aib-HGTFTSDYSKYLDEK(1)AAKDFVQWLLDQHPSSGQPPPS |
| 21 | Y-Aib-QGTFTSDYSKYLDEK(1)RAKDFVQWLLDQHPSSGQPPPS |
| 22 | Y-Aib-QGTFTSDYSKYKDEK(2)AAKEFVQLLDHHPSSGQPPPS |
| 23 | Y-Aib-QGTFTSDYSKYLDEK(2)RAKEFVQWLLDHHPSSGQPPPS |
| 24 | Y-Aib-QGTFTSDYSKYLDEK(2)AAKDFVQWLLDQHPSSGQPPPS |
| 25 | Y-Aib-QGTFTSDYSKYLDEK(2)RAKDFVQWLLDHHPSSGQPPPS |
| 26 | Y-Aib-QGTFTSDYSEALDEK(2)RAKEFQWLLDQHPSSGQPPPS |
| 27 | H-Aib-HGTFTSDYSKYLDEK(2)AAKDFVQWLLDQHPSSGQPPPS |
| 28 | Y-Aib-QGTFTSDYSKYLDEK(2)RAKDFVQLLDQHPSSGQPPTS |

In Table 1, K(1) and K(2) refer to the acylated amino acid K(1) and K(2) having the above structures, respectively. The underlined amino acids (glutamic acid at position 16 and lysine at position 20) mean that they form a lactam bridge with each other.

The prepared triple agonists were purified using reverse-phase chromatography. The purity of the synthesized peptides was confirmed using analytical liquid chromatogram (RP-HPLC), and when the purity was 90% or more, it was considered to be useful for experimentation. In addition, the molecular weight and information of the peptides were confirmed using liquid chromatogram/mass spectrometry (LC/MS).

The synthesized peptides were stored at -20°C until used in experiments.

### Experimental Example 1: Confirmation of in vitro Activity of Triple Agonists

In order to measure the activities of the triple agonists prepared in Example above, a method of measuring *in vitro* cellular activities using cell lines, where a GLP-1 receptor, a GIP receptor, and a glucagon (GCG) receptor are each transformed, was used.

Each of the cell lines above is one in which the genes for a human GLP-1 receptor, a human GCG receptor and a human GIP receptor are transformed into Chinese hamster ovary (CHO), respectively, to be expressed therein, and is thus suitable for the measurement of the activities of GLP-1, GCG and GIP. Accordingly, the activity for each part was measured using the respective transformed cell lines.

For the measurement of the GLP-1 receptor activity of the triple agonists prepared in Example above, human GLP-1 was subjected to a 3-fold serial dilution from 4 nM to 0.00007 nM, and the triple agonists of SEQ ID NOS: 1 to 9 were subjected to a 3-fold serial dilution from 40 nM to 0.00068 nM. The culture solution was removed from the cultured CHO cells, in which the human GLP-1 receptor was expressed, and each of the serially diluted materials was added to the CHO cells in an amount of 10 µL, respectively, and cultured at room temperature for 15 minutes. Then, an Eu-cAMP tracer mix and anti-cAMP detection mix containing a cell lysis buffer were sequentially added thereto in an amount of 5 µL for the lysis of the cells and reacted at room temperature for 60 minutes under light-shielded conditions. The cAMP accumulated in the cell lysates, after completion of the reaction, was measured using fluorescence, and the EC₅₀ value was calculated from the accumulated cAMP, and the values were compared with one another. The relative titers relative to human GLP-1 are shown in Table 2 below.

For the measurement of the GCG (glucagon) receptor activity of the triple agonists prepared in Example above, human GCG was subjected to a 3-fold serial dilution from 4 nM to 0.00007 nM, and the triple agonists of SEQ ID NOS: 1 to 9 were subjected to a 3-fold serial dilution from 40 nM to 0.00068 nM. The culture solution was removed from the cultured CHO cells, in which the human GCG receptor was expressed, and each of the serially diluted materials was added to the CHO cells in an amount of 10 µL, respectively, and cultured at room temperature for 15 minutes. Then, an Eu-cAMP tracer mix and anti-cAMP detection mix containing a cell lysis buffer were sequentially added thereto in an amount of 5 µL for the lysis of the cells and reacted at room temperature for 60 minutes under light-shielded conditions. The cAMP accumulated in the cell lysates, after completion of the reaction, was measured using fluorescence, and the EC₅₀ value was calculated from the accumulated cAMP, and the values were compared with one another. The relative titers relative to human GCG are shown in Table 2 below.

For the measurement of the GIP receptor activity of the triple agonists prepared in Example above, human GIP was subjected to a 3-fold serial dilution from 4 nM to 0.00007 nM, and the triple agonists of SEQ ID NOS: 1 to 9 were subjected to a 3-fold serial dilution from 40 nM to 0.00068 nM. The culture solution was removed from the cultured CHO cells, in which the human GIP receptor was expressed, and each of the serially diluted materials was added to the CHO cells in an amount of 10 µL, respectively, and cultured at room temperature for 15 minutes. Then, an Eu-cAMP tracer mix and anti-cAMP detection mix containing a cell lysis buffer were sequentially added thereto in an amount of 5 µL for the lysis of the cells and reacted at room temperature for 60 minutes under light-shielded conditions. The cAMP accumulated in the cell lysates, after completion of the reaction, was measured using fluorescence, and the EC₅₀ value was calculated from the accumulated cAMP, and the values were compared with one another. The relative titers relative to human GIP are shown in Table 2 below.

**[Table 2]**

| **Relative Titer Ratio of Triple Agonists** | | | |
|---|---|---|---|
| | In vitro Activity Compared to Native Peptide (%) | | |
| SEQ ID NO: | vs. GLP-1 | vs. Glucagon | vs. GIP |
| 1 | 5.88 | 0.33 | 5.18 |
| 2 | 3.87 | 3.35 | 2.17 |
| 3 | 0.02 | 0.40 | 7.04 |
| 4 | 3.71 | 2.36 | 3.81 |
| 5 | 2.36 | 1.51 | 5.29 |
| 6 | 7.04 | 1.36 | 6.11 |
| 7 | 4.21 | 3.32 | 5.26 |
| 8 | 9.68 | 5.89 | 4.89 |
| 9 | 14.99 | 0.51 | 8.42 |

Based on the results, it was confirmed that the triple agonists of SEQ ID NOS: 1 to 9 of the present disclosure prepared in the Example above have activities for all of three GLP-1, GIP, and glucagon receptors.

### Experimental Example 2: Weight Loss Effect by Triple Agonists in Obese Mice Induced by High-Fat, High-Fructose, and High-Cholesterol Diet

In order to confirm the weight loss effect of each of the three triple agonists of SEQ ID NO: 1, SEQ ID NO: 2, and SEQ ID NO: 4 prepared in the Example above, an AMLN (amylin) mouse model, known as an obesity model, was used. AMLN diet-induced mice were divided into an excipient control group and groups administered with three types of triple agonists (351 ug/kg, Q2D, subcutaneously), and the administration was repeated for 2 weeks.

After repeated administration for 2 weeks, the body weight was measured, and the change in body weight relative to the body weight immediately before administration (D0) was measured to evaluate weight loss effect.

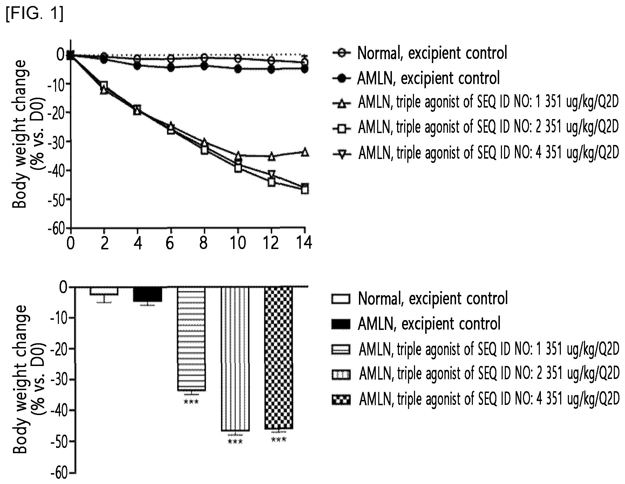

As a a result, when the triple agonist of SEQ ID NO: 1, the triple agonist of SEQ ID NO: 2, and the triple agonist of SEQ ID NO: 4 were administered repeatedly for 2 weeks, a significant weight loss efficacy relative to the excipient control group was confirmed (FIG. 1, p<0.001 vs. excipient control by one-way ANOVA).

### Experimental Example 3: Weight Loss Effect by Triple Agonists in Obese Mice Induced by High-Fat Diet

In order to confirm the weight loss effect of each of the four triple agonists of SEQ ID NO: 3, SEQ ID NO: 5, SEQ ID NO: 6, and SEQ ID NO: 7 prepared in the Example above, a DIO mouse model, known as an obesity model, was used. DIO diet-induced mice were divided into an excipient control group and groups administered with the triple agonists (60 nmol/kg, Q2D, subcutaneously), and the administration was repeated for 2 weeks.

After repeated administration for 2 weeks, the body weight was measured, and the change in body weight relative to the body weight immediately before administration (D0) was measured to evaluate weight loss effect.

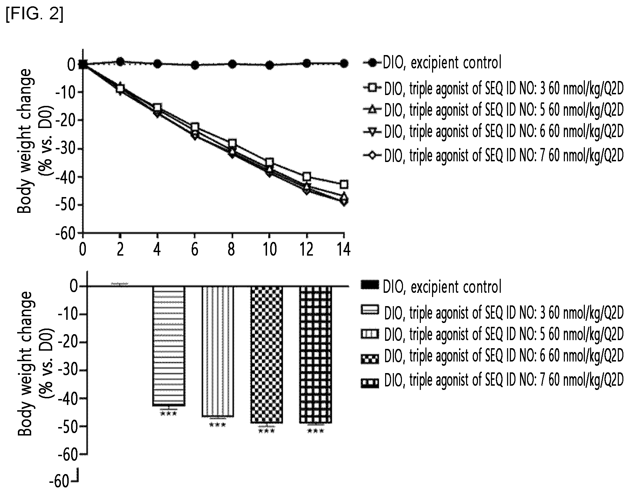

As a a result, when the four long-acting conjugates of the triple agonists of the present disclosure were administered repeatedly for 2 weeks, a significant weight loss efficacy relative to the excipient control group was confirmed (FIG. 2, p<0.001 vs. excipient control by one-way ANOVA).

From the Examples above, it was confirmed that the triple agonists prepared in the present disclosure can act on the GLP-1 receptor, GIP receptor, and glucagon receptor, and thus can be used as an effective therapeutic agent for the treatment of obesity.

From the foregoing, a skilled person in the art to which the present disclosure pertains will be able to understand that the present disclosure may be embodied in other specific forms without modifying the technical concepts or essential characteristics of the present disclosure. In this regard, the exemplary embodiments disclosed herein are only for illustrative purposes and should not be construed as limiting the scope of the present disclosure. On the contrary, the present disclosure is intended to cover not only the exemplary embodiments but also various alternatives, modifications, equivalents, and other embodiments that may be included within the spirit and scope of the present disclosure as defined by the appended claims.

## Claims

1. A peptide having activities for a glucagon-like peptide-1 (GLP-1) receptor, a glucose-dependent insulinotropic polypeptide (GIP) receptor, and a glucagon receptor, the peptide comprising an amino acid sequence represented by General Formula 1 below:
X1-Aib-X3-G-T-F-T-S-O-Y-S-X12-X13-L-O-E-X17-X18-A-K-X21-F-V-Q-W-L-L-D-X29-H-P-S-S-G-Q-P-P-P-S (General Formula 1, SEQ ID NO: 29)
in General Formula 1 above,
X1 is histidine or tyrosine;
X3 is glutamine or histidine;
X12 is lysine or an acylated amino acid;
X13 is α-methyl-leucine, tyrosine, or alanine;
X17 is lysine or an acylated amino acid;
X18 is alanine or arginine;
X21 is aspartic acid or glutamic acid; and
X29 is histidine or glutamine;
wherein - represents a peptide bond, and the peptide does not contain cysteine.

2. The peptide of claim 1, wherein the peptide has higher relative activities for the GIP receptor and GLP-1 receptor relative to native GIP and GLP-1, respectively, compared to the relative activity for the glucagon receptor relative to native glucagon.

3. The peptide of claim 2, wherein the peptide has 4 times or higher relative activities for the GIP receptor and GLP-1 receptor relative to native GIP and GLP-1, respectively, compared to the relative activity for the glucagon receptor relative to native glucagon.

4. The peptide of claim 1, wherein, in General Formula 1 above, X3 is histidine.

5. The peptide of claim 1, wherein, in General Formula 1 above, X13 is tyrosine; and X18 is alanine.

6. The peptide of claim 1, wherein, in General Formula 1 above, X1 is tyrosine; X3 is glutamine; X13 is tyrosine; and X18 is arginine.

7. The peptide of claim 1, wherein, in General Formula 1 above, X29 is glutamine.

8. The peptide of claim 1, wherein an acyl group is attached to one or more amino acids of the peptide through a linker selected from a group consisting of (2-(2-aminoethoxy)ethoxy)acetic acid (AEEA), 4-aminobutyric acid (GABA), 5-aminovaleric acid (Ava), aminohexanoic acid (Ahx), triazole, and polyethylene glycol (PEG).

9. The peptide of claim 8, wherein the linker comprises AEEA.

10. The peptide of claim 8, wherein the linker comprises 0 to 3 AEEA.

11. The peptide of claim 1, wherein the acylated amino acid is an amino acid represented by K(1) or K(2) below:
[K(1)] C₂₀ diacid-gammaGlu-(AEEA)₂-Lys 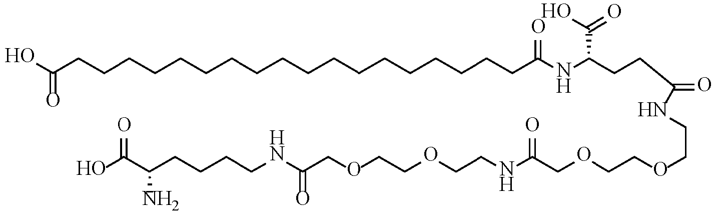
[K(2)] C₁₈ diacid-gammaGlu-(AEEA)₂-Lys

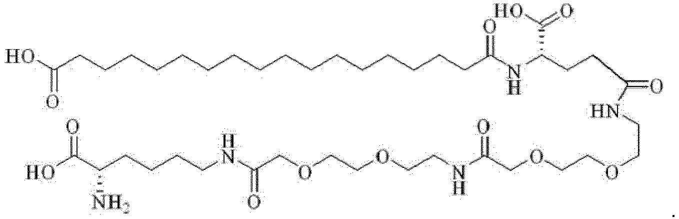

12. The peptide of claim 1, wherein the peptide is amidated at the C-terminus.

13. The peptide of claim 1, wherein the peptide has any one structure from (i) to (iv) below:

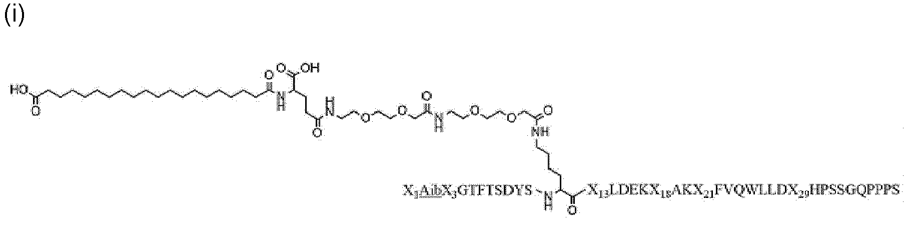

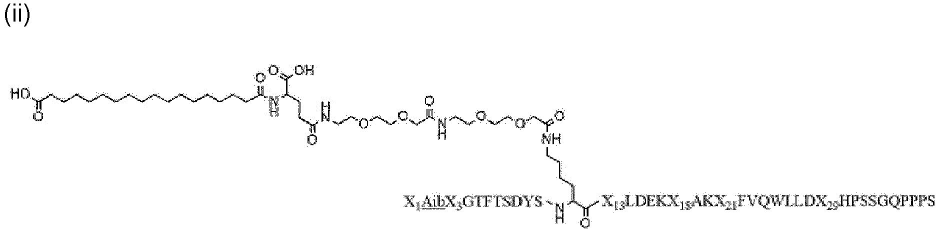

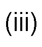

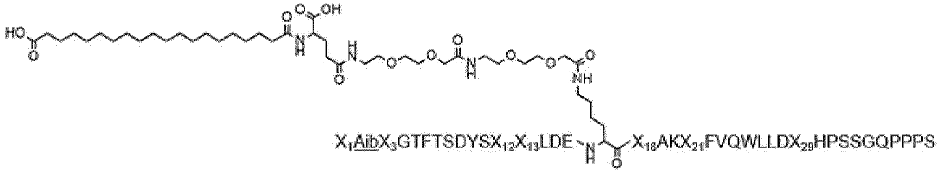

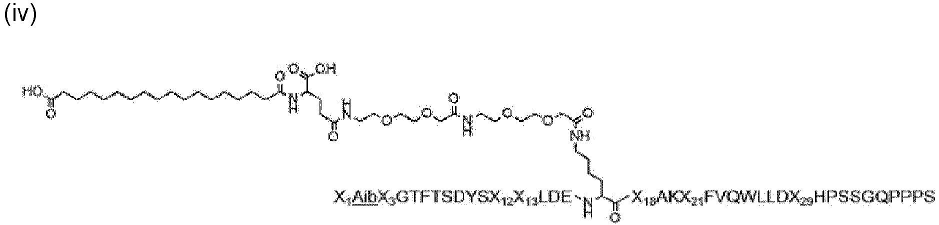

14. The peptide of claim 1, wherein the peptide comprises any one sequence selected from the group consisting of amino acid sequences of SEQ ID NOS: 1 to 28.

15. The peptide of claim 1, wherein the amino acids at positions 16 and 20 from the N-terminus of General Formula 1 form a ring with each other.

16. The peptide of claim 1, wherein the peptide has the structure of General Formula 2 below: 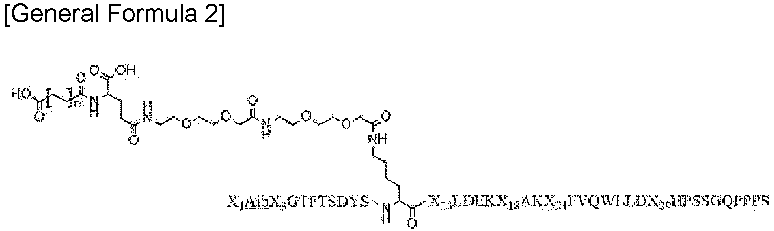 in General Formula 2 above,
n is 16 or 18;
X1 is histidine or tyrosine;
X3 is glutamine or histidine;
X13 is α-methyl-leucine, tyrosine, or alanine;
X18 is alanine or arginine;
X21 is aspartic acid or glutamic acid; and
X29 is histidine or glutamine.

17. A pharmaceutical composition for preventing or treating obesity, comprising the peptide of any one of claims 1 to 16 in a pharmaceutically effective amount.

18. The pharmaceutical composition of claim 17, wherein the pharmaceutical composition further comprises a pharmaceutically acceptable carrier.

19. The pharmaceutical composition of claim 17, wherein the pharmaceutical composition has an effect of reducing the body weight of an individual upon administration.

20. The pharmaceutical composition of claim 17, wherein the peptide has any one structure from (i) to (iv) below:

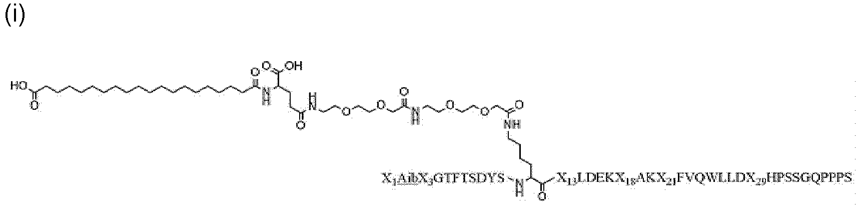

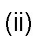

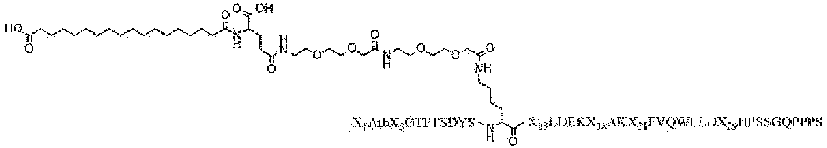

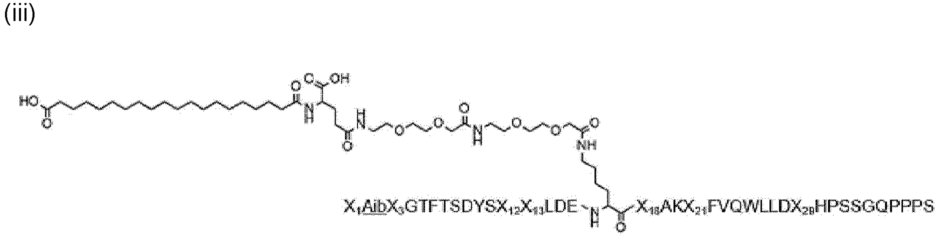

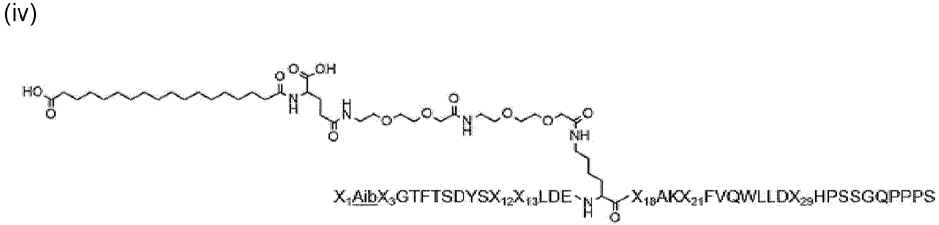

21. The pharmaceutical composition of claim 17, wherein the peptide has the structure of General Formula 2 below: 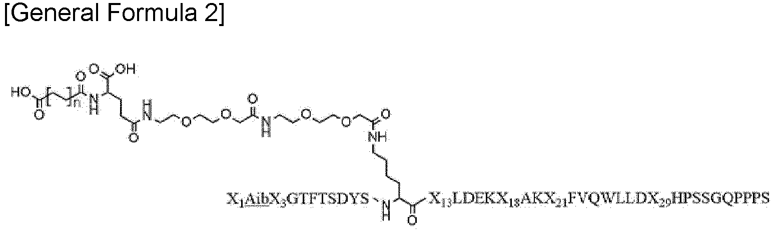 in General Formula 2 above,
n is 16 or 18;
X1 is histidine or tyrosine;
X3 is glutamine or histidine;
X13 is α-methyl-leucine, tyrosine, or alanine;
X18 is alanine or arginine;
X21 is aspartic acid or glutamic acid; and
X29 is histidine or glutamine.
